(19) Europäisches Patentamt
European Patent Office
Office européen des brevets

(11) Veröffentlichungsnummer : **0 150 433**
**B1**

(12) **EUROPÄISCHE PATENTSCHRIFT**

(45) Veröffentlichungstag der Patentschrift : **30.12.86**

(51) Int. Cl.⁴ : **C 07 C 45/68**, C 07 C 49/217, C 07 C 79/36

(21) Anmeldenummer : **84115685.4**

(22) Anmeldetag : **18.12.84**

(54) **Verfahren zur Herstellung ungesättigter Ketone.**

(30) Priorität : **27.01.84 DE 3402732**

(43) Veröffentlichungstag der Anmeldung : **07.08.85 Patentblatt 85/32**

(45) Bekanntmachung des Hinweises auf die Patenterteilung : **30.12.86 Patentblatt 86/52**

(84) Benannte Vertragsstaaten : **CH DE FR GB LI**

(56) Entgegenhaltungen :
**THE JOURNAL OF ORGANIC CHEMISTRY, Band 22, Januar-Juni 1957, Seiten 41-45, Mack Printing Co., US; F. RAMIREZ et al.: "Phosphinemethylenes. II. Triphenylphosphineacylmethylenes"**
**JOURNAL OF THE CHEMICAL SOCIETY CHEM. COMM., 1981, Seiten 556-557; S.V. ATTWOOD et al.: "Model studies on the synthesis of milbemycin beta3"**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Becker, Rainer, Dr.**
**Im Haseneck 22**
**D-6702 Bad Duerkheim (DE)**
Erfinder : **Theobald, Hans, Dr.**
**Queichstrasse 6**
**D-6703 Limburgerhof (DE)**
Erfinder : **Jahn, Dieter, Dr.**
**Burgunder Weg 8**
**D-6803 Edingen-Neckarhausen (DE)**
Erfinder : **Keil, Michael, Dr.**
**Fontanestrasse 4**
**D-6713 Freinsheim (DE)**
Erfinder : **Schirmer, Ulrich, Dr.**
**Berghalde 79**
**D-6900 Heidelberg (DE)**

**EP 0 150 433 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung α,β-ungesättigter Ketone (Vinylketone). Vinylketone sind wichtige Zwischenprodukte z. B. bei der Synthese von Wirkstoffen, wie Herbiziden (DE-OS 3 123 312) oder anderen Zielprodukten für deren Herstellung die Struktur —CH=CH—CO— benötigt wird.

Die bekannten Verfahren zur Synthese α,β-ungesättigter Ketone beruhen z. B. auf Umsetzung entsprechender Aldehyde RCHO :

(A) $R-CH=O + CH_3COCH_3 \longrightarrow R-CH=CH-CO-CH_3$ (Aldol-Kondensation)

(B) $R-CH=O + \underset{COOR}{CH_2}-CO-CH_3 \longrightarrow \longrightarrow R-CH=CH-CO-CH_3$ (Knoevenagel-Reaktion)

(C) $R-CH=O + Ø_3P=CHCOCH_3 \longrightarrow R-CH=CH-CO-CH_3$ (Wittig-Reaktion)

Eine Variante der Knoevenagel-Kondensation mit dem Na-Salz der Acetessigsäure anstelle des Acetessigesters wird in der DE-OS 3 117 271 beschrieben.

Das neue Verfahren vermeidet nun den nach den bekannten Verfahren erforderlichen Aldehyd RCHO. Dies ist vor allem bei der Synthese heterocyclisch substituierter Vinylketone von Bedeutung, da die entsprechenden Aldehyde oft gar nicht oder nur schwierig herstellbar sind.

Die Erfindung beruht auf der Überlegung, anstelle dieser Aldehyde die aus der entsprechenden Halogenmethylverbindung und Triphenylphosphin leicht zugänglichen Wittig-Ylide (II) mit einem geeigneten Aldehyd umzusetzen nach der Gleichung

$$R-CH_2-\overset{\oplus}{P}Ø_3 \text{ (II) } Cl^{\ominus} + HCOCOCH_3 \text{ (III) } \xrightarrow{\text{Base}} R-CH=CH-CO-CH_3 \quad \text{(I)}$$

Bringt man die Ylide (II) mit wäßrigem Methylglyoxal (III) zur Reaktion, so erhält man die gewünschten ungesättigten Ketone in sehr guter Ausbeute. Dieser glatte Reaktionsverlauf ist vor allem deshalb überraschend, weil bekanntlich Phosphorylide in wäßrig-alkalischer Umgebung zur Hydrolyse neigen und weil außerdem das Methylglyoxal unter diesen Bedingungen zur Eingenkondensation neigt.

Die Ausführung der Wittig-Reaktion im wäßrigen Medium ist an sich, z. B. in Form der Wittig-Horner-Reaktion unter Verwendung der Phosphonate beschrieben, welche beispielsweise im Zweiphasensystem Natronlauge/Benzol mit aromatischen Aldehyden zu Stilbenderivaten reagieren (Synthesis *1974*, 869 und *1976*, 187) :

$$Aryl-CH_2-PO(OR)_2 + Aryl^{*}-CHO \longrightarrow Aryl-CH=CH-Aryl^{*}$$

Diese Umsetzung läßt sich jedoch bemerkenswerterweise mit Methylglyoxal anstelle von z. B. Benzaldehyd nicht ausführen. Setzt man nämlich unter sonst gleichen Bedingungen wäßriges Methylglyoxal anstelle des aromatischen Aldehyds ein, so wird das Phosphonesterderivat unverändert zurückgewonnen ; auch die Verschärfung der Bedingungen (Temperaturerhöhung) führt nicht zur Umsetzung.

Ebenso läßt sich die in Syn. Comm. *1976*, 53 beschriebene Reaktion eines Phosphorylids mit wäßrigem Formaldehyd unter Zugabe einer Base nicht auf das System Methylglyoxal/Wasser übertragen.

Bei Kenntnis der der Erfindung am nächsten kommenden Umsetzungen muß es daher als ausgesprochen überraschend angesehen werden, daß bei Verwendung der im Vergleich zu Benzylphosphonestern weniger reaktiven Benzyltriphenylphosphoniumchloride die Umsetzung mit wäßrigem Methylglyoxal glatt zu Methylvinylketonen I führt. Ein ebenso problemloser Reaktionsverlauf wird bei Einsatz von heterocyclisch substituierten Methyltriphenylphosphoniumchloriden beobachtet.

R ist z. B. entweder ein Phenylrest oder ein substituierter Phenylrest, wobei der Substituent ein- oder mehrfach vorhanden sein und z. B. Halogen, nieder-Alkyl, Nitro, Cyano, Halogenalkyl mit 1 bis 4 C-Atomen ; Alkoxy, Halogenalkoxy, Alkylthio, Alkylsulfinyl, Alkylsulfonyl und deren halogensubstituierte Derivate mit jeweils 1 bis 4 C-Atomen, ferner Amino, alkyl- oder acylsubstituiertes Amino sein kann.

Es versteht sich, daß die Bedeutung des Verfahrens für Verbindungen, in denen R Phenyl oder substituiertes Phenyl ist, gering sein kann, weil im Einzelfall der zugehörige Aldehyd leicht zugänglich ist und dann die Zielprodukte güngstig mittels Aldolkondensation erhalten werden können.

Größere Bedeutung hat das Verfahren für 6-gliedriges und insbesondere 5-gliedriges heterocyclisches, quasi-aromatisches R. Dessen Aldehyde sind meist nur schwer oder gar nicht zugänglich.

Typische Bedeutungen für heterocyclisches R sind daher z. B. die unten angeführten.

Geeignete Ausgangsstoffe für das erfindungsgemäße Verfahren sind demnach Phosphorylide II, in denen der Substituent R z. B. ein ggf. beliebig substituierter Arylrest ist, wie Phenyl, Naphthyl, Chlorphenyl, Fluorphenyl, Trifluormethylphenyl, Nitrophenyl, Tolyl, Methoxyphenyl, Methylthiophenyl, Ethylthiophenyl, Methylsulfinylphenyl, Ethylsulfinylphenyl, Methylsulfonylphenyl, Ethylsulfonylphenyl, Ethylphenyl.

Besonders wichtig sind solche Ylide, bei denen R ein ggf. beliebig substituierter Heterocyclus ist wie z. B. Isoxazolyl, Methylisoxazolyl, Ethylisoxazolyl, iso-Propylisoxazolyl, Phenylisoxazolyl, Methoxymethylisoxazolyl, Methoxycarbonylisoxazolyl, Thiadiazolyl, Methylthiazolyl, Oxazolyl, Methyloxazolyl, Thienyl, Furyl, Pyrryl, Pyrazolyl, Imidazolyl, Triazolyl, Pyridyl, Methylpyridyl, Ethylpyridyl, Chlorpyridyl, Fluorpyridyl, Pyrimidyl, Methylpyrimidyl, Pyrazinyl, Pyrridazyl, Methylthiadiazolyl, Methyloxadiazolyl.

Zu Verbindungen, die das Strukturelement zwei- oder gar mehrfach enthalten, gelangt man natürlich dann, wenn man von entsprechenden, mehrwertigen Phosphoryliden ausgeht, die im Prinzip ebenfalls leicht zugänglich sind.

Die Umsetzung wird zweckmäßigerweise in zweiphasiger (Wasser/Lösungsmittel-)Umgegung durchgeführt. Als mit Wasser nicht bzw. begrenzt mischbares Lösungsmittel sind geeignet Aromaten wie Benzol, Toluol, Xylol ; chlorierte Kohlenwasserstoffe wie Methylenchlorid, Chloroform, Dichlorethan oder Ether wie Diethylether, Methyltertiärbutylether.

Die Umsetzung verläuft i. a. oberhalb von 0 °C ausreichend schnell, meistens bei 20 bis 50 °C. Sie wird ausgelöst z. B. durch Alkalihydroxid, -carbonat oder -hydrogencarbonat bzw. entsprechende Erdalkaliverbindungen. Das Molverhältnis von Phosphorylid zum Alkali beträgt 1 : 1, Methylglyoxal wird in stöchiometrischer Menge eingesetzt oder, da es im allgemeinen der billigste Bestandteil sein dürfte, in einem gewissen Überschuß.

Die Konzentrationsangaben in den nachstehenden Beispielen beziehen sich auf das Gewicht des Gelösten, d. h. sie bedeuten (g/100 ml Lösung).

## Beispiel 1

179 g 3-Methyl-5-isoxazolylmethyl-triphenylphosphoniumchlorid werden in 1 000 ml Wasser aufgenommen und mit 108 g 40 %iger wäßriger Lösung von Methylglyoxal sowie mit 500 ml Toluol versetzt. Anschließend werden 38 g festes Natriumhydrogencarbonat in Portionen eingetragen.

Nach 20 Stunden Rühren wird die Toluolphase abgetrennt, mit Wasser gewaschen und eingeengt. Der verbleibende Rückstand wird mit Cyclohexan/Ether ausgerührt und liefert nach Absaugen und Einengen des Filtrats 66 g β-(3-Methylisoxazol-5-yl)-vinylmethylketon als gelbes Öl ; aus dem NMR-Spektrum ergibt sich ein cis-trans-Verhältnis von 6 : 4 (Gesamtausbeute 97 %, bezogen auf Ylid). Durch Säulenchromatographie läßt sich die trans-Verbindung in reiner Form isolieren, Fp. 78 bis 79 °C.

| NMR-Spektrum | cis | trans |
|---|---|---|
| $CO-C\underline{H}_3$ | δ = 2,30 ppm | 2,35 |
| $-C\underline{H}=C\underline{H}-$ | 6,68 } AB-System<br>6,40 } Koppl 13 Hz | 6,68 } AB-System<br>7,30 } Koppl 16 Hz |
| 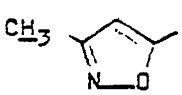 | 2,30 | 2,30 |
| 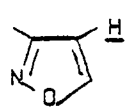 | 7,20 | 6,36 |

## Beispiel 2

43,4 g p-Nitrobenzyltriphenylphosphoniumchlorid werden in 200 ml Wasser suspendiert und mit 28,8 g 50 %iger wäßriger Lösung von Methylglyoxal sowie mit 150 ml Toluol versetzt. Anschließend werden 8,4 g festes Natriumhydrogencarbonat in Portionen eingetragen.

Nach 20 Stunden Rühren bei Raumtemperatur wird die organische Phase abgetrennt, mit Wasser gewaschen und eingeengt. Der verbleibende Rückstand enthält neben dem gewünschten Produkt noch Triphenylphosphinoxid ; durch Ausrühren mit Cyclohexan/Ether kann dieses als unlöslicher Rückstand abfiltriert werden. Das Filtrat liefert nach Abziehen des Lösungsmittels 13,4 g p-Nitrobenzylidenaceton als cis/trans-Gemisch im Verhältnis 3 : 1 (Ausbeute 68 %).

| NMR-Spektrum | cis | trans |
|---|---|---|
| $CO-CH_3$ | δ = 2,23 ppm | 2,39 |
| -CH=CH- | 6,39 } AB-System<br>6,87 } Koppl 13 Hz | 6,81 } AB-System<br>~7,6 } Koppl 17 Hz |
| 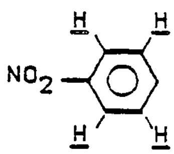 | 7,67 } AB-System<br>8,17 } Koppl 9 Hz | 7,7 } AB-System<br>8,2 } Koppl 9 Hz |

Beispiel 3

42,6 g Pyrid-2-ylmethyltriphenylphosphoniumchlorid × HCl werden in 200 ml Wasser aufgenommen und mit 28,8 g 50 %iger wäßriger Lösung von Methylglyoxal sowie mit 150 ml Toluol versetzt. Anschließend werden 16,8 g Natriumhydrogencarbonat portionsweise zugegeben. Nach mehrstündigem Rühren wird die Toluolphase abgetrennt, gewaschen und eingeengt. Der Rückstand wird in Cyclohexan/Ether verrührt und abgesaugt ; das Filtrat ergibt nach Abziehen des Lösungsmittels 13,4 g β-(Pyrid-2yl)vinylmethylketon als cis-trans-Gemisch im Verhältnis 1 : 9 (Ausbeute 91 %).

| NMR-Spektrum | cis | trans |
|---|---|---|
| $CO-CH_3$ | δ = 2,25 ppm | 2,32 |
| -CH=CH- | 6,12 } AB-System<br>6,62 } Koppl 12 Hz | 7,0 } AB-System<br>7,43 } Koppl 16 Hz |
| 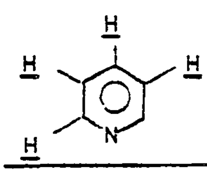 | 7,1 - 8,6 (m) | 7,1 - 8,6 (m) |

Nach den vorstehenden Angaben können aus entsprechenden Ylid-Verbindungen die in der nachstehenden Tabelle aufgeführten substituierten Vinylketone als cis/trans-Gemische hergestellt werden.

(Siehe Tabelle Seite 5 ff.)

Tabelle

| Beispiel Nr. | chem. Bezeichnungsweise | | NMR-Spektrum cis | NMR-Spektrum trans |
|---|---|---|---|---|
| 4 | ß-(4-Methyl-1,2,3-thiadiazol-5-yl-) vinylmethylketon | -COCH3 | δ = 2,35 ppm (s) | 2,39 (s) |
| | | -CH=CH- | 6,57 / 7,00 } AB-System, Koppl 12 Hz | 6,63 / 7,5 } AB-System, Koppl 16 Hz |
| | | 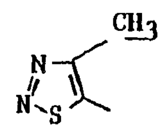 | 2,74 (s) | 2,78 (s) |
| 5 | ß-(3-iso-Propylisoxazol-5-yl-) vinylmethylketon | -COCH3 | 2,35 | 2,38 |
| | | -CH=CH- | 6,38 / 6,61 } AB-System, Koppl 12 Hz | 6,75 / 7,31 } AB-System, Koppl 16 Hz |
| | | 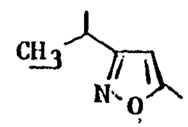 | - 1,3 (d) - | |
| | | 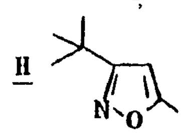 | - 3,09 (quintett) | |
| | | 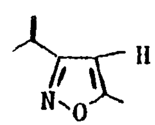 | | |

| Beispiel Nr. | chem. Bezeichnungsweise | | NMR-Spektrum cis | trans |
|---|---|---|---|---|
| 6 | ß-Phenylvinylmethylketon (Benzylidenaceton) | $-CO-CH_3$ | 2,10 (s) | 2,30 (s) |
| | | $-CH=CH-$ | 6,08 } 6,76 } AB-System Koppl 13 Hz | 6,60 } 7,25 } AB-System Koppl 17 Hz |
| | | 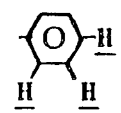 | - 7,3 (m) - | |
| 7 | ß-(2-iso-Propyl-1,3,4-oxadiazol-5-yl-)vinylmethylketon | $COCH_3$ | - 2,42 (s) - | |
| | | $-CH=CH-$ | 6,57 (s) | 6,98 } 7,38 } AB-System Koppl 16 Hz |
| | | 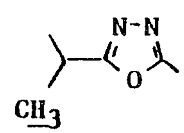 | 1,35 (d) | 1,44 (d) |
| | | 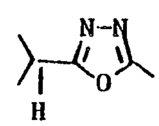 | - 3,2 (m) - | |
| 8 | ß-(2-Methyl-1,3,4-thiadiazol-5-yl-)vinylmethylketon | $COCH_3$ | | 2,38 (s) |
| | | $-CH=CH-$ | | 6,73 } 7,61 } AB-System Koppl 16 Hz |
| | | 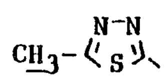 | | 2,76 (s) |

**Patentansprüche**

1. Verfahren zur Herstellung ungesättigter Ketone (Vinylketone) der allgemeinen Struktur (I)

$$R{-}CH{=}CH{-}CO{-}CH_3 \qquad I,$$

wobei R einen beliebigen, ggf. substituierten aromatischen oder heterocyclischen Rest bedeutet, dadurch gekennzeichnet, daß ein entsprechendes phosphor-Ylid (II)

$$R{-}CH_2{-}\overset{\oplus}{P}\emptyset_3 \quad Cl^{\ominus} \qquad (II)$$

mit einer wäßrigen Lösung von Methylglyoxal in Gegenwart einer Base umgesetzt wird.

2. Verfahren nach Anspruch 1, dadurch gekennzeichnet, daß R einen heterocyclischen Rest bedeutet, ausgewählt aus der Gruppe, die umfaßt Isoxazolyl, Methylisoxazolyl, Ethylisoxazolyl, iso-Propylisoxazolyl, Phenylisoxazolyl, Methoxymethylisoxazolyl, Methoxycarbonylisoxazolyl, Thiadiazolyl, Methylthiazolyl, Oxazolyl, Methyloxazolyl, Thienyl, Furyl, Pyrryl, Pyrazolyl, Imidazolyl, Triazolyl, Pyridyl, Methylpyridyl, Ethylpyridyl, Chlorpyridyl, Fluorpyridyl, Pyrimidyl, Methylpyrimidyl, Pyrazinyl, Pyrridazyl, Methylthiadiazolyl, Methyloxadiazolyl.

3. Verfahren nach Anspruch 1, dadurch gekennzeichnet, daß die Umsetzung in Gegenwart eines mit Wasser nicht oder nur begrentz mischbaren Lösungsmittels vorgenommen wird.

**Claims**

1. A process for the preparation of an unsaturated ketone (vinyl ketone) of the general formula (I)

$$R{-}CH{=}CH{-}CO{-}CH_3 \qquad I,$$

where R is any unsubstituted or substituted aromatic or heterocyclic radical, wherein an appropriate phosphorylid (II)

$$R{-}CH_2{-}\overset{\oplus}{P}\emptyset_3 \quad Cl^{\ominus} \qquad (II)$$

is reacted with an aqueous solution of methylglyoxal in the presence of a base.

2. A process as claimed in claim 1, wherein R is a heterocyclic radical selected from the group consisting of isoxazolyl, methylisoxazolyl, ethylisoxazolyl, isopropylisoxazolyl, phenylisoxazolyl, methoxymethylisoxazolyl, methoxycarbonylisoxazolyl, thiadiazolyl, methylthiazolyl, oxazolyl, methyloxazolyl, thienyl, furyl, pyrryl, pyrazolyl, imidazolyl, triazolyl, pyridyl, methylpyridyl, ethylpyridyl, chloropyridyl, fluoropyridyl, pyrimidyl, methylpyrimidyl, pyrazinyl, pyrridazyl, methylthiadiazolyl and methyloxadiazolyl.

3. A process as claimed in claim 1, wherein the reaction is carried out in the presence of a solvent which is waterimmiscible or exhibits only limited miscibility with water.

**Revendications**

1. Procédé de préparation de cétones insaturées (vinyl-cétones) de la structure générale (I)

$$R{-}CH{=}CH{-}CO{-}CH_3 \qquad I,$$

où R représente un reste aromatique ou hétérocyclique, quelconque, éventuellement substitué, caractérisé par le fait que l'on fait réagir, en présence d'une base, un ylure de phosphore (II) correspondant

$$R{-}CH_2{-}\overset{\oplus}{P}\emptyset_3 \quad Cl^{\ominus} \qquad (II)$$

avec une solution aqueuse de méthylglyoxal.

2. Procédé selon la revendication 1, caractérisé par le fait que R représente un reste hétérocyclique, choisi dans le groupe comprenant isoxazolyle, méthylisoxazolyle, éthylisoxazolyle, iso-propylisoxazolyle, phénylisoxazolyle, méthoxyméthylisoxazolyle, méthoxycarbonylisoxazolyle, thiadiazolyle, méthylthiazolyle, oxazolyle, méthyloxazolyle, thiényle, furyle, pyrryle, pyrazolyle, imidazolyle, triazolyle, pyridyle, méthylpyridyle, éthylpyridyle, chloropyridyle, fluoropyridyle, pyrimidyle, méthylpyrimidyle, pyrazinyle, pyrridazyle, méthylthiadiazolyle, méthyloxadiazolyle.

3. Procédé selon la revendication 1, caractérisé par le fait que la réaction est effectuée en présence d'un solvant non miscible ou seulement de miscibilité limitée avec l'eau.